(19) Europäisches Patentamt
European Patent Office
Office européen des brevets

(11) EP 0 818 207 A2

(12) **EUROPEAN PATENT APPLICATION**

(43) Date of publication:
**14.01.1998 Bulletin 1998/03**

(51) Int Cl.[6]: **A61L 29/00**

(21) Application number: **97304227.8**

(22) Date of filing: **17.06.1997**

(84) Designated Contracting States:
**AT BE CH DE DK ES FI FR GB GR IE IT LI LU MC NL PT SE**

(30) Priority: **21.06.1996 GB 9613010**

(71) Applicant: **The BOC Group plc**
**Windlesham Surrey GU20 6HJ (GB)**

(72) Inventor: **Elliott, Thomas Stuart Jackson**
**Sutton Coldfield, West Midlands, B74 3AE (GB)**

(74) Representative: **Wickham, Michael et al**
**c/o Patent and Trademark Department**
**The BOC Group plc**
**Chertsey Road**
**Windlesham Surrey GU20 6HJ (GB)**

(54) **Medical article**

(57) A medical article, particularly a central venous catheter, releasably incorporates a first therapeutically acceptable antibacterial agent comprising a quaternary ammonium compound other than a quaternary ammonium salt of an amino acid or other amine substituted organic acid and a second therapeutically acceptable antibacterial agent comprising an amino acid or other amine substituted organic acid or an acid addition salt of the amino acid or the other amine substituted organic acid. The first antibacterial agent is preferably benzalkonium chloride and the second antibacterial agent is preferably L-arginine or L-arginine hydrochloride.

EP 0 818 207 A2

**Description**

This invention relates to a medical article; particularly an article such as a catheter intended for the administration of a fluid to a human being or an animal.

Catheters, particularly central venous catheters, are widely used in medical care, their clinical applications including central venous pressure monitoring, drug administration, fluid replacement and total parenteral nutrition. Unfortunately, such catheters are associated with a number of complications including early and late onset infections in relation to the time of catheter insertion. Resulting infections vary from localised thrombophlebitis to septicaemia, a hazard of intravenous therapy that complicates a significant proportion of venous cannulations, and can be localised at the insertion site or, in the example of septicaemia, systemic. There are four routes by which organisms may gain access to the intravascular segment of the catheter. These are extraluminal, intraluminal, by haematogenous spread or via contaminated infusates, the first two of these routes being predominant. The main sources of organisms related to catheter sepsis are the skin insertion site and the catheter hub.

Many bacterial and fungal species are related to catheter sepsis. The micro-organisms most commonly implicated in central venous catheter - related infections are the coagulase - negative staphylococci, particularly Staphylococcus epidermis. These micro-organisms are gram-positive skin commensals which have been recognised as opportunistic pathogens when associated with implanted vascular devices. Gram-negative micro-organisms, particularly Pseudomonas aeruginosa, are, however, also associated with not an insignificant number of cases of sepsis resulting from catherisation of a patient.

It has, therefore, been proposed to coat surfaces of a central venous catheter with an antibacterial agent. According to EP-A-520 160 the antibacterial agent is a benzalkonium halide (preferably benzalkonium chloride). Other quaternary ammonium compounds are also known to have anti-bacterial activity. Benzalkonium chloride is hydrophilic and exhibits in vitro activity primarily against Gram-positive cocci (for example, Staphylococcus aureus), and to a lesser degree, Gram-negative bacteria and Candida albicans. Benzalkonium chloride is a suitable antimicrobial agent for the incorporation into a polymeric central venous catheter because it is not routinely used for the treatment of infections (it has however been proposed in WO-A-95/30414 to incorporate benzalkonium chloride into an ocular care solution for the storage of contact lenses. An acid or neutral amino acid may also be employed in the solution to enhance its antimicrobial activity and a basic amino acid to reduce irritancy). Hence the possible emergence of resistant organisms is unlikely to make therapeutic use of the catheter more difficult in the future. Further, benzalkonium chloride has a broad antimicrobial spectrum, is able to retain its activity in the catheter in vitro for up to at least seven days, and has no local or systemic toxicity.

These are, however, two drawbacks to benzalkonium chloride and other quaternary ammonium antibacterial agents. First, its activity against the gram-negative micro-organism, Pseudomonas Aeruginosa is limited, particularly at the concentrations which can readily be incorporated into a central venous catheter. Secondly, there is a substantial reduction in its activity in the presence of human plasma. Indeed, it has been observed that there is a sixteen fold reduction in activity in the presence of a 25% solution of human plasma. Therefore, benzalkonium chloride is less effective in vivo than in vitro.

It is an aim of the present invention to provide a medical article which is able to lessen the above drawbacks.

According to the present invention, there is provided a medical article releasably incorporating a first therapeutically acceptable antibacterial agent comprising a quaternary ammonium compound other than a quaternary ammonium salt of an amino acid or other amine substituted organic acid and a second therapeutically acceptable antibacterial agent comprising an amino acid or other amine substituted organic acid or an acid addition salt of the amino acid or the other amine substituted organic acid.

The medical article preferably comprises a member of plastics material. The medical article preferably has at least one passage therethrough suited for therapeutic use in the administration of fluids to a human being or animal or in the withdrawal of a body fluid from the human being or animal. The medical article may therefore be a cannula or catheter, particularly a central venous catheter, or a device, particularly if formed of plastics material, which may be associated in use with the catheter or cannula, for example a Luer lock.

The plastics material is preferably a therapeutically acceptable thermoplastic polyurethane. Examples of preferred therapeutically acceptable polyurethanes are polytetramethylene ether glycol diphenylmethane diisocyanate ("MDI"), polytetramethylene ether glycol toluene diisocyanate ("TDI"), poly (1,4 - oxybutylene) glycol diphenyl methane diisocyanate, polypropylene glycol toluene diisocyanate, polyethylene adipate diphenyl methane diisocyanate, and polyethylene polypropylene adipate isophorone isocyanate polyurethanes, and the like, and mixtures thereof. A particularly preferred therapeutically acceptable polyurethane is PELLETHANE (Registered Trade Mark) Series 2363 polyurethane which is a polyether polyurethane synthesised from methylene bis - (p-phenyl isocyanate) polytetramethylene glycols and 1,4 - butanediol and is commercially available from Dow Chemical Company.

The polyurethane or other plastics member preferably has a coating comprising a hydrophilic layer which become lubricious when it comes into contact with body fluids. The layer is preferably of a therapeutically acceptable homopolymer or copolymer of an N-vinyl lactam, for example, N-vinylpyrrolidone, N-vinylbutyrolactam, N-vinylcaprolactam, or a mixture of two or more of the homopolymers or copolymers. A preferred N-vinyl lactam polymer is a polyvinyl pyrrolidone (PVP) homopolymer having a weight average molecular weight in the range of 1,000,000 to 1,200,000.

The layer is preferably formed by applying to the plastics member a solution comprising from 1 to 10%, preferably 2.5 to 5%, by weight of N-vinyl lactam polymer or polymers in a suitable organic solvent. The solvent is preferably a mixture of methyl ethyl ketone, ethyl lactate and trichloroethylene. The plastics member may be contacted with the solution by conventional methods such as dipping and spraying. Excess coating solution is removed and the coated plastics member is dried in an oven at elevated temperature so as to drive off any residual solvent.

There are various different ways of incorporating the antibacterial agents into the plastics member. For example, one or both of the antibacterial agents may be mixed with a chosen monomer prior to polymerisation. The resulting polymer is formed into the desired shape either during polymerisation or thereafter. In another example, one or both of the antibacterial agents are mixed with a chosen polymer before curing. In a further example, one or both antibacterial agents are combined with a chosen polymer and extruded to form a chosen medical article. By utilising different polymer mixtures containing varying amounts of antibacterial agent, shaped medical articles, such as catheters, can be formed by coextrusion techniques with a plurality of layers containing different antibacterial agents or different concentrations of antibacterial agents. In general, all such examples are not preferred since exposure of the antibacterial agents, particularly benzalkonium halides, to the necessary high temperatures and pressures can lead to their degradation.

It is also possible to apply to the plastics member, one or both of the antibacterial agents prior to applying, as a coating, the layer that becomes lubricious when it comes into contact with body fluids. Such examples are also not preferred since the presence of the antibacterial agent or agents may compromise the bond between the plastics member and the coating which becomes lubricious when it comes into contact with body fluids.

It is therefore preferred to impregnate the coated plastics member with the first and second antibacterial agents. The antibacterial agents are preferably applied by immersing the plastics member or at least one chosen part of it in a solution of the first and second bacterial agents, removing the catheter from the solution, and drying the catheter. The solution is preferably aqueous. Typically, the solution contains, in total, from 1 to 10% by weight of the antibacterial agents, more typically from 2 to 5% by weight. Typically, the immersion time is from 5 to 60 seconds. Preferred values of these parameters depend on the dimensions of the member to be coated and on the amount of each antibacterial agent which it is desired to add to the plastics member, bearing in mind that, for example, in the case of a central venous catheter, if the weight of antibacterial agents per unit length of the catheter is too low, the rate of release of antibacterial agent in vivo may be too low to be efficacious clinically, but if the weight of antibacterial agent per unit length of catheter is too high, the rate of release of antibacterial agent may be sufficiently high for adverse medical effects such as mild haemolysis to occur. Within these limits there is sufficient flexibility to be able readily to select coating time and concentrations of impregnating solution so as to enable there to be made an antimicrobial catheter which is able to release the antibacterial agents at an effective rate in vivo without causing any haemolysis.

Alternatively, the finished plastics member may be impregnated first with one of the anti-bacterial agents and then with the other. The first anti-bacterial agent is typically applied first. After being impregnated with the anti-bacterial agents the plastics member is preferably dried in a humid atmosphere at a temperature of up to 70°C. If the medical article is a catheter or cannula, it is preferably impregnated with the first and second antibacterial agents from both internal and external surfaces. It is particularly important that the impregnated solution comprising the first and second antibacterial agents be applied to that length of the catheter which in use is inserted into a patient (i.e. the distal end) and to its hub.

The first antibacterial agent may be any quaternary ammonium compound (other than a quaternary salt of as amino acid or other amine substituted organic acid) known to have anti-bacterial activity. Such quaternary ammonium compounds include cetyldimethylethyl ammonium halides, particularly cetyldimethylethyl ammonium bromide, and hexadecyltrimethyl ammonium halides, particularly hexadecyltrimethyl ammonium bromide, and benzethonium halides, particularly benzethonium chloride. The preferred first antibacterial agent is, however, one or more therapeutically acceptable unsubstituted benzalkonium salts preferably of the formula (I):

Formula I

$$[C_6H_5.CH_2.N.(CH_3)_2.CnH_{2n+1+}]^+X^-$$

where n is an integer from 8 to 18, and X is a therapeutically acceptable anion, preferably a halide, more preferably a chloride. Preferred benzalkonium halides according to formula I comprise tetradecyl dimethyl benzyl ammonium halides, hexadecyl dimethyl benzyl ammonium halides, and octadecyl dimethyl benzyl ammonium halides, particularly the chlorides thereof.

Alternatively, if desired, the benzyl group of formula I may contain one or more substituents. For example, the

benzalkonium salt may be an alkyl dimethyl dichlorobenzyl ammonium salt, particularly an alkyl dimethyl dichlorobenzyl ammonium halide.

The second antibacterial agent preferably comprises a basic amino acid or a therapeutically acceptable addition salt thereof. Suitable basic amino acids comprise lysine, arginine, hydroxylysine, ornithine and histidine. Arginine and its therapeutically acceptable addition salts (for example, halides) are particularly preferred.

The second antibacterial agent may alternatively or additionally comprise a neutral amino acid or a therapeutically acceptable acid addition salt thereof. Suitable neutral amino acids comprise glycine, alcinine, valine, leucine, isoleucine, methionine, proline, hydroxyproline, asparagine, glutamine, phenylalanine, tyrosine, tryptophan, serine and threonine.

The second antibacterial agent may alternatively or additionally comprise an amine substituted acetic, tartaric, oxalic, or citric acid, or a therapeutically acceptable addition salt thereof. For example, the second antibacterial agent may comprise a salt of ethylenediamine tetraacetic acid (EDTA), particularly disodium ethylene diamine tetraacetic acid.

Preferably the impregnating solution containing the second antibacterial agent has a pH in the range of 7.0 to 9.5 more preferably from 7.5 to 9.5. If necessary, a suitable buffering agent may be added to the impregnating solution so as to keep its pH at a desired value.

It has been found that the second antibacterial agent enhances the effectiveness in vitro of the first antibacterial agent both against Gram-positive and Gram-negative micro-organisms. It has further been found that the second antibacterial agent also enhances the effectiveness of the first antibacterial agent against Gram-negative micro-organisms in the presence of human plasma. Indeed, a synergistic interaction between the first and second antibacterial agents takes place rendering medical articles, particularly central venous catheters, according to the invention particularly effective in clinical use in that they provide effective prophylaxis against both Gram-positive and Gram-negative micro-organisms.

The invention will now be further illustrated by the following Examples.

## Example 1

In the experiments to be described below there were used 18 control lengths, each of 2cm, of a commercial central venous catheter formed of polyurethane with a hydrophilic polyvinylpyrrolidone (PVP) homopolymer coating and a further 18 lengths, each of 2cm, of the same central venous catheter but with the coating impregnated with a commercial benzalkonium chloride composition of formula:

$$[C_6H_5 - CH_2 - NR. (CH_3)_2]^+ Cl^-$$

in which in 50% by weight of the benzalkonium chloride R was a $C_{12}H_{25}$ group; in 30% by weight in $C_{14}H_{29}$ group; in 17% by weight a $C_{16}H_{33}$ group, and in 3% by weight a $C_{18}H_{37}$ group.

In each experiment three lengths of catheter were vertically suspended in nutrient broth containing Pseudomonas aeruginosa ($5 \times 10^5$ cfu/ml). After incubation for one hour at 37°C, the lengths of catheter were removed and washed eight times in a phosphate buffered saline solution. The washing removed any bacteria not adhering to the catheter. The lengths of catheter were then resuspended in a nutrient broth. In half the examples, the broth contained 0.5% by weight of L-arginine. In the other half, the broth was free of L-arginine. After incubation for three hours, at 37°C, the catheters were removed and washed eight times in a phosphate buffered saline solution. The number of organisms remaining on the catheter surface was determined by rolling each catheter length three times across the surface of a CLED (cystine-lactose-electrolyte deficient) agar plate. After incubation for 24 hours at 37°C, the number of colonies was counted.

Four sets of experiments were conducted with respectively;

a) a control catheter in arginine - free broth;
b) a control catheter in an arginine - containing broth;
c) an impregnated catheter in an arginine - free broth;
d) an impregnated catheter in an arginine containing broth.

Each set of experiments was performed using three lengths of catheter and was repeated two further times.

The results set out in Table 1 were obtained.

Table 1

| Experiment | No of cfu/2cm length of catheter (Mean ± Standard Deviation, n = 9) |
|---|---|
| a | Confluent growth (> 1000) |
| b | 522 ± 153 |
| c | 407 ± 96 |
| d | 45 ± 4 |

The results show that both benzalkonium chloride alone (compare experiment b with experiment a) and L-arginine alone (compare experiment c with experiment a) have some antibacterial effect in relation to Pseudomonas aeruginosa, but that in combination the two antibacterial agents have a substantially stronger effect (compare experiment d with the other experiments).

The pH of the arginine free broth was 7.5 and that of the arginine containing broth was 9.4.

**Example 2**

The effect of L-arginine on the minimum inhibitory concentration (MIC) of benzalkonium chloride (BZC) against Pseudomonas aeruginosa was investigated employing standard microbiological methods. The MIC is the lowest concentration of an antibacterial agent required to inhibit bacterial growth in vitro. The results are set out in Table 2 below:

Table 2

| Addition to BZC | MIC (mg/ml) | Decrease in MIC |
|---|---|---|
| None | 50 | - |
| 2% arginine | 3 | 16 fold |
| 0.5% arginine | 12.5 | 4 fold |
| 0.25% arginine | 25 | 2 fold |
| 0.125% arginine | 25 | 2 fold |

In Table 2, the addition to BZC is set out in terms of the percentage by weight of arginine in the BZC solution.

The results in Table 2 show that the combination of L-arginine and benzalkonium chloride is particularly effective against Pseudomonas aeruginosa.

**Example 3**

The effect of L-arginine on the minimum inhibitory concentration (MIC) of benzalkonium chloride (BZC) against Staphylococcus epidermidis was investigated employing standard microbiological methods. The results are set out in Table 3 below.

| Addition to BZC | BZC MIC (ng/ml) | Decrease in MIC |
|---|---|---|
| None | 780 | - |
| 2% arginine | <12 | >64 fold |
| 0.5% arginine | <12 | >64 fold |
| 0.25% arginine | 48 | 16 fold |
| 0.125% arginine | 96 | 8 fold |
| 0.015% arginine | 192 | 4 fold |

In Table 3, the addition to BZC is set out in terms of the percentage by weight of arginine in the arginine solution.

The results in Table 3 show that L-arginine in combination with benzalkonium chloride is particularly effective against Staphylococus epidermis, a prevalent Gram-positive micro-organism.

**Example 4**

The minimum inhibitory concentration (MIC) and minimum bactericidal concentration (MBC) of BZC and L-arginine hydrochloride against Pseudomonas aeruginosa by a standard doubling broth dilution microtitre plate method in the absence and presence of 25% by volume of human plasma. The MBC is the lowest concentration of an antibacterial agent that kills 99.9% of the bacteria being tested. In some of the experiments, there was planktonic growth of the bacteria; in other experiments the bacteria were cultured as a biofilm on a substrate for a given period of time ("the adherence period") before incubation in the presence of a chosen antibacterial agent and in the presence or absence of human plasma.

The results are set out in Table 4 below. It was found that L-arginine hydrochloride depressed the pH of the culture broth from 7.5 to about 7.1. In one set of experiments potassium hydroxide was added to restore the pH to 7.5.

Table 4

| Antibacteria l agent | concentration of human plasma | Type of bacterial growth | Incubation period | MIC (mg/ml) | MBC (mg/ml) |
|---|---|---|---|---|---|
| BZC | - | planktonic | 24h | 78 | 78 |
| BZC | 25% | planktonic | 24h | 625 | not measured |
| BZC | - | biofilm | 24h adherence 24h incubation | 130 | 1250 |
| L-arginine HCl | - | planktonic | 24h | 160 | 160 |
| L-arginine | 25% | planktonic | 24h | 80 | 160 |
| L-arginine HCl + KOH | - | planktonic | 24h | 80 | 80 |
| L-arginine HCl | - | biofilm | 24h adherence24h incubation | 160 | >160 |

The results show that the MIC of BZC is substantially increased in the presence of human plasma and if the Pseudomonas aeruginosa are caused to grow as a biofilm.

However, the MIC of L-arginine hydrochloride is substantially unaffected by these two factors.

It was further noted that increasing the pH of the L-arginine hydrochloride broth, and thereby forming a mixture of L-arginine and L-arginine hydrochloride, reduced its MIC. Thus, mixtures of L-arginine and a therapeutically acceptable acid addition salt of L-arginine may be particularly useful as the second antibacterial agent.

**Example 5**

Benzalkonium chloride (BZC) - impregnated lengths and non-impregnated lengths of catheter were made. The BZC had the same composition as in Example 1. The catheters were of the same length and composition as those used in Example 1. 100ml BHI (brain heart infusion) broth, or 75ml BHI and 25ml human plasma, were inoculated with an overnight culture of a test strain of Pseudomonas aeruginosa to establish a final concentration of 1 x $10^6$ organisms/ml, the number of organisms having been so selected that between 200 to 300 cfu became attached to the control catheters during the incubation period. Three lengths of BZC-impregnated catheter were then introduced into the broth culture. An identical container was established but the BZC catheters were substituted with three control CVCs. Both containers were aerobically incubated at 37°C with shaking at 200 rpm for 2 hours or 24 hours to allow the micro-organisms to adhere to the catheter surface. Following incubation the catheters were removed from the broth and washed by slowly inverting 40 times in each of eight aliquots of 10ml sterile PBS (pH 7.2) (phosphate buffered saline), in order to remove loosely attached organisms but not those colonising the catheter. A roll plate catheter culture technique as described in Example 1 was adopted to make a semi-quantitative estimation of the numbers of viable organisms colonising the external catheter surface. Each catheter was rolled across the surface of a CLED agar plate three times. After 24 hours of incubation at 37°C in air the number of colony forming units (cfus) was counted.

Sets of experiments were performed in the presence and absence of L-arginine hydrochloride. Experiments with L-arginine hydrochloride were performed at 0.5 MIC and 0.1 MIC concentrations. In some experiments KOH was added to the broth in order to restore its pH to 7.5. In other experiments there was no KOH addition. In two sets of experiments employing L-arginine hydrochloride at 0.5 MIC concentration, the broth was supplemented with human plasma at 25 volume % concentration.

The results are set out in Tables 5, 6 and 7 below. Table 5 identifies the bacterial adherence to catheters after a 2 hour colonisation period and a further four hour exposure to BHI broth alone or to BHI broth supplemented with L-arginine hydrochloride. Table 6 identifies the bacterial adherence to catheters after a 24 hour colonisation period and a further 4 hour exposure to BHI broth alone or BHI broth supplemented with L-arginine hydrochloride. Table 7 identifies the bacterial adherence to catheters after a 24 hour colonisation period and a further 24 hour exposure to BHI broth alone, BHI broth supplemented with human plasma (a 25% by volume solution), BHI broth supplemented with L-arginine hydrochloride, and BHI broth supplemented with both human plasma (a 25% by volume solution) and L-arginine hydrochloride.

TABLE 5

| Antibacterial Agent (ABA) | Antibacterial agent concentration | Mean number of organisms (cfu) | | | |
|---|---|---|---|---|---|
| | | Control catheter No ABA | Control catheter + ABA | BZC catheter No ABA | BZC catheter + ABA |
| L-arginine HCl | 0.5 MIC (80 mg/ml) | >1000 | 199 | 654 | 198 |
| L-arginine HCl (+ KOH; pH7.5) | 0.5 MIC (80 mg/ml) | 602.5 | 250 | 279.5 | 29 |
| L-arginine HCl | 0.1 MIC (16 mg/ml) | 807 | >1000 | 467 | 975 |

TABLE 6

| Antibacterial Agent (ABA) | Antibacterial agent concentration | Mean number of organisms (cfu) | | | |
|---|---|---|---|---|---|
| | | Control catheter No ABA | Control catheter + ABA | BZC catheter No ABA | BZC catheter + ABA |
| L-arginine HCl | 0.5 MIC (80 mg/ml) | >1000 | 915 | 744 | 286 |
| L-arginine HCl | 0.1 MIC (16 mg/ml) | >1000 | >1000 | 802 | >1000 |

TABLE 7

| Antibacterial Agent (ABA) | Antibacterial agent concentration | Mean number of organisms (cfu) | | | |
|---|---|---|---|---|---|
| | | Control catheter No ABA | Control catheter + ABA | BZC catheter No ABA | BZC catheter + ABA |
| L-arginine HCl | 0.5 MIC (80 mg/ml) | >1000 | >1000 | 961 | 28 |
| L-arginine HCL (in presence of 25% human plasma) | 0.5 MIC (80 mg/ml) | >1000 | 43 | >1000 | 1 |

TABLE 7 (continued)

| Antibacterial Agent (ABA) | Antibacterial agent concentration | Mean number of organisms (cfu) | | | |
|---|---|---|---|---|---|
| | | Control catheter No ABA | Control catheter + ABA | BZC catheter No ABA | BZC catheter + ABA |
| L-arginine HCl | 0.1 MIC (16 mg/ml) | >1000+ | >1000+ | >1000+ | >1000 |

Tables 5 to 7 demonstrate that BZC at a given impregnation level is not particularly effective by itself against Pseudomonas aeruginosa. When L-arginine hydrogen chloride was used at 0.1 MIC concentration in conjunction with the BZC-impregnated catheter there was little beneficial effect in the absence of human plasma. When however L-arginine was added at 0.5 MIC concentration, there was a clear enhancement of antibacterial activity both in the absence and presence of human plasma. At short incubation periods in the absence of human plasma the greater reduction in bacterial colonisation was produced by arginine hydrochloride alone. However, at incubation periods of 24h with a further exposure of 24h to arginine hydrochloride, the combination of this agent with BZC was found to be for more effective than arginine hydrochloride alone. The results in human plasma are particularly good and are clearly indicative of synergy between the two antibacterial agents.

The mechanism of the antibacterial activity of BZC and other quaternary ammonium upon a microbial cell is multiple and has been attributed to the inactivation of energy producing oxidative enzymes, e.g. the glucose, succinate and pyruvate systems, denaturation of essential cell proteins and disruption of the cell membrane. BZC is believed to act on the surface of the bacterial cell. Because bacteria themselves are particulate, they also act to adsorb BZC. Adsorption onto the cell is followed by penetration into the cell wall leading to its disruption. The mechanism whereby BZC interferes with microbial colonisation of a central venous catheter is that it exerts its effect directly on the organisms closely aligned to the polymer surface, possibly also limiting the attachment process and thereby reducing colonisation rather than leaching out and lessening the numbers. Despite the activity of BZC against Pseudomonas aeruginosa generally, this agent was not found to be by itself highly efficient at affording protection of the catheter from colonisation by this micro-organism. A combination of factors may contribute to the reduced effect of BZC. Nonetheless, much of the comparative resistance of Gram-negative rod-shaped bacteria such as Pseudomonas aeruginosa to antibacterial agents such as BZC is probably a consequence of the complex nature of the permeability barrier presented by the outer membrane unique to this group of organisms.

BZC is known to be most effective against micro-organisms at neutral or slightly alkaline pH and becomes virtually inactive below pH 3.5. The results obtained on elevation of the pH confirm this knowledge. The slight increase of only 0.48 pH units was enough significantly to enhance the antibacterial action of BZC and a combination of the latter with arginine hydrochloride produced a large effect in reducing the numbers of Pseudomonas aeruginosa adhering to the catheters at the elevated pH.

The results obtained with human plasma suggest that the activity of BZC was reduced in the presence of protein. In contrast, no reduction effect of human plasma on the actions of arginine hydrochloride was seen. Because of the amphoteric chemical nature of amino acids, they are believed not to be deactivated by proteins. Further, the results obtained with human plasma indicate that BZC-impregnated catheters did not lose any antimicrobial activity and therefore suggest that arginine hydrochloride through its amphoteric nature interferes in some way with the linkage of BZC to serum protein whilst still enabling itself to retain and express an antibacterial effect. Since all basic and neutral amino acids have basic and acidic groups, other basic amino acids (or their acid addition salts) or a neutral amino acid may be substituted for L-arginine hydrochloride. It is also clear from Example 1 that L-arginine itself rather than its acid addition salt is also effective in the present invention.

A method of preparing a catheter according to the invention is set out in Example 6 below.

## Example 6

A triple lumen, 14 gauge, tubular member, 20cm in length, of Dow Pellethane 2363 polyurethane is given a polyvinylpyrrolidone (PVP) coating over essentially its entire length by immersion in a solution of the PVP containing 2.8% by weight of solids. (The solvent comprises a mixture of methyl ethylketone, trichloroethylene and ethyl lactate.) The polyurethane member is held immersed in the coating solution for sixty seconds at 25°C and a pressure of 1 bar. The resulting coated member is dried in an oven for 1 hour at a temperature of 55°C.

The resulting coated catheter is then immersed in a diluted aqueous solution of STEPAN BTC 50 USP (trade mark)

(available from Stepan Company, Northfield, Illinois 60093, United States of America) antimicrobial agent containing 3% by weight of active ingredients to which is added 2% by weight of L-arginine hydrochloride. The solution is buffered at pH 7.5 The active ingredients of the benzalkonium chloride all of formula:

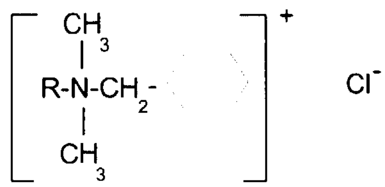

where R was a higher alkyl group. In 50% by weight of the active ingredients R was a $C_{12}H_{25}$ group; in 30% a $C_{14}H_{29}$ group; in 17% a $C_{16}H_{33}$ group, and in 3% a $C_{18}H_{37}$ group. The catheter is held immersed for 30 seconds at 25°C and 1 bar. The resulting benzalkonium chloride/L-arginine hydrochloride impregnated catheter is dried in an hour for 1 hour at a temperature of 55°C and a pressure of 1 bar. The resulting catheter was found to have taken up 194 microgrammes of benzalkonium chloride/cm of the catheter.

The impregnated catheter is then sterilised in a chamber for four hours in a humid atmosphere of a sterilising gas mixture consisting of 75% by volume of ethylene oxide and 25% by volume of carbon dioxide at a temperature of 50% by volume of carbon dioxide at a temperature of 50°C and a pressure of 85 kPa. (The humidity is present in the atmosphere for only three and a quarter hours of the four hour sterilising period.)

## Claims

1. A medical article releasably incorporating a first therapeutically acceptable antibacterial agent comprising a quaternary ammonium compound other than a quaternary ammonium salt of an amino acid or other amine substituted organic acid and a second therapeutically acceptable anti-bacterial agent comprising an amino acid or other amine substituted organic acid or an acid addition salt of the amino acid or the other amine substituted organic acid.

2. A medical article as claimed in claim 1, in which the first anti-bacterial agent comprises at least one therapeutically acceptable benzalkonium salt.

3. A medical article as claimed in claim 2, in which the benzalkonium salt is of the formula:

$$[C_6H_5 . CH_2 . N^+(CnH_{2n+1}) (CH_3)_2]^+ X^-$$

where n is an integer from 8 to 18, and X is a therapeutically acceptable anion.

4. A medical article as claimed in claim 3, in which X is chloride.

5. A medical article as claimed in claim 2, in which the benzalkonium salt is an alkyl dimethyl dichlorobenzyl ammonium salt.

6. A medical article as claimed in any one of the preceding claims, in which the second anti-bacterial agent comprises one or both of arginine, and a therapeutically acceptable addition salt thereof.

7. A medical article as claimed in any one of claims 1 to 5, in which the second anti-bacterial agent comprises (a) a basic amino acid selected from one or more of lysine, hydroxylygine, ornithine and histidine and therapeutically acceptable acid addition salts thereof, (b) a neutral amino acid selected from one or more of glycine, alanine, valine, leucine, isoleucine, methionine, proline, hydroxyproline, asparagine, glutamine, phenylalanine, typosine, tryptophane, serine, and threonine, and therapeutically acceptable acid addition salts thereof; (c) an amine substituted acetic, tartaric, oxalic or citric acid, or a therapeutically acceptable addition thereof; or (d) disodium ethylenediamine tetraacetic acid.

8. A medical article as claimed in any one of the preceding claims, comprising plastics material and having at least

one passage therethrough for therapeutic use in the administration of fluids to a human being or an animal or in the withdrawal of a body fluid.

**9.** A medical article as claimed in any one of the preceding claims, being a Luer lock, a cannula or a catheter.

**10.** A medical article as claimed in claim 9, in which the catheter is a central venous catheter and has a coating comprising a hydrophilic layer which becomes lubricious when it comes into contact with body fluids.

**11.** A method of making a medical article as claimed in claim 10, comprising immersing the catheter or at least one chosen part of it in an aqueous solution of the first and second anti-bacterial agents, removing the catheter from the solution, and drying the catheter.

**12.** The use of an amino acid or a therapeutically acceptable acid addition salt thereof in making an anti-bacterial composition by mixing the amino acid or salt thereof with a first anti-bacterial agent comprising a quaternary ammonium compound (other than a quaternary ammonium salt of an amino acid or other amine substituted organic acid), the first anti-bacterial agent being impregnated into a catheter.